# EUROPEAN PATENT APPLICATION

(11) **EP 4 606 355 A1**
(43) Date of publication of application: **27.08.2025**
(21) Application number: 24158955.5
(22) Date of filing: 21.02.2024
(51) Int. Cl.: A61F 2/60, A61F 2/68

(54) **ACTIVE HIP DISARTICULATION PROSTHESIS**

(71) Applicant: Université catholique de Louvain, 1348 Louvain-la-Neuve (BE)
(72) Inventor: DEVILLEZ, Louis, 1348 Louvain-la-Neuve (BE); RONSSE, Renaud, 1348 Louvain-la-Neuve (BE); HERMAN, Benoît, 1348 Louvain-la-Neuve (BE)
(74) Representative: Icosa

(57) **Abstract**

The present invention relates to a system (100) for an external hip disarticulation prosthesis comprising an angled planar double parallelogram mechanism, an actuator (60) configured to fold the double parallelogram mechanism and a femoral stem (50) fixed to the double parallelogram mechanism and configured to rotate around a remote rotation center (40), the double parallelogram mechanism comprising an anchoring element (30) configured to provide a unique connection with a receiving shell (400).

## Description

### FIELD OF INVENTION

The present invention relates to a system for a hip disarticulation prosthesis. More precisely, to an active system for an external hip disarticulation prosthesis.

### BACKGROUND OF INVENTION

Hip disarticulation is the highest amputation level of the lower-limb.

Pelvis prostheses have been developed to provide a replacement of the whole lower limb of the amputees without surgical intervention. The main components of such a prosthesis are a receiving shell fitting to the stump, and a femoral segment comprising a femoral stem connected to a knee module.

Several passive prostheses are known. Although they offer an efficient solution for amputees, they also induce several important complications due either to the bone luxation or to the prosthesis itself (material fracture, component dislocation, etc...). Furthermore, despite being sophisticated and expensive, the passive prostheses do not sufficiently restore normal biomechanical functions. In particular, their passive nature prevents them to replicate the mechanical energy production of a sound hip during the gait.

Other protheses are equipped with a power actuator. Some of these active protheses advantageously allow to remotely place the center of rotation of the femoral stem inside the receiving shell. The center of rotation is remotely placed thanks to a double parallelogram mechanism comprised in the femoral segment. Therefore, when worn by a user, the center of rotation of the femoral stem is located inside the user's body, preferably close to the hip natural center of rotation. One example of such a double parallelogram mechanism is disclosed in S. Luo, X. Shu, H. Zhu, and H. Yu, "Design and optimization of a new integrated hip and knee prosthesis structure", Artificial Organs, vol. 2023, July 2023 (hereafter S. Luo et al.).

However, the known active prostheses with a remote center of rotation are bulky. Indeed, to place the remote rotation center close to the hip natural center of rotation, the double parallelogram mechanism of the femoral segment needs to be joined on the front of the receiving shell close to its edge. Therefore, the double parallelogram mechanism protrudes forward the user's body leading to unaesthetic aspect of the prosthesis.

Moreover, the double parallelogram mechanism of the known prosthesis needs a second passive joint with the receiving shell to provide constraints and supports for the stability in the stance phase of the gait. This second passive joint is located on the underside of the receiving shell therefore providing discomfort when the user is sitting. Moreover, this second passive joint implies to modify the structure of the known receiving shells which provide only an anchoring position on the front of the shell.

There is thus a need for an active prosthesis with a remote center of rotation which is compact, which does not protrude forward the user's body, which is compatible with the known receiving shells and/or which provides a comfortable sitting position to the user - *i.e.,* with clearance under the shell. In addition, such an active prosthesis should preferably be able to extend over a human-like range, from normal gait to sitting position.

To this end, a purpose of this invention is to provide a system comprising an angled planar double parallelogram mechanism defining a remote rotation center close to the hip natural center of rotation, an actuator configured to fold the double parallelogram mechanism, the double parallelogram mechanism comprising an anchoring element configured to provide a unique connection with a receiving shell.

### SUMMARY

This invention thus relates to a system for an external hip disarticulation prosthesis comprising:
- A double parallelogram mechanism comprising four singular links and two common links, each of the singular links and common links comprising two extremities,
   the two common links joining together at a common rotation axis, the common rotation axis being positioned between the extremities of each common link thereby dividing each common link into two shared links, and
   for at least one common link, the shared links of said common link being angled by a fixed non-zero inclination angle,
- An actuator configured to fold the double parallelogram mechanism around the common rotation axis, and
- A femoral stem fixed to one of the singular links,

the double parallelogram mechanism having a planar kinematics,
the double parallelogram mechanism comprising an anchoring element configured to provide a unique connection with a receiving shell.

Indeed, thanks to the angled common link, the remote rotation center is brought up with respect to the double parallelogram mechanism compared to the prior art systems. The anchoring element may thus be anchored closer to the bottom of the receiving shell thereby bringing the double parallelogram mechanism towards the back of the user. Therefore, the double parallelogram mechanism fits the volume of the natural leg of the user. The system therefore does not protrude forward the user's body when anchored to a receiving shell and worn by the user. Moreover, the compacity of the system is thus increased. In addition, the angle of the vertex formed by the shared links defining a secondary virtual parallelogram is larger than 90° to allow the complete folding of the double parallelogram mechanism until the sitting configuration. Finally, the unique connection of the anchoring element with the receiving shell provides a comfortable sitting position to the user thanks to a clear space bellow the receiving shell when the system is in a sitting configuration.

According to other advantageous aspect of the invention, the femoral stem has a femoral angle with the singular link to which it is fixed, the femoral angle being a fixed angle ranging from 10° to 80°.

This allows to provide a vertical femoral stem when the double parallelogram mechanism is in a standing configuration.

According to other advantageous aspect of the invention, the unique connection between the anchoring element and the receiving shell is a rigid connection.

According to other advantageous aspect of the invention, for each angled common link, the fixed inclination angle is ranging from 10° to 80°.

Indeed, when the inclination angle is less than 10°, the double parallelogram mechanism protrudes in the front of the user's body when the system is worn by the user. When the inclination angle is larger than 80° the double parallelogram mechanism cannot be folded up to the sitting configuration wherein the femoral stem has an angle of 90° with the direction of the gravity.

According to other advantageous aspect of the invention, the actuator is configured to provide a peak absolute torque ranging from 100 Nm to 200 Nm.

According to other advantageous aspect of the invention, the actuator is configured to provide, when the system is worn by a user, mechanical energy during a gait of the user.

This allows to help the user during the gait. Indeed, the force that must be generated by the user to step the prothesis forward is decreased by the torque generated by the actuator.

According to other advantageous aspect of the invention, the actuator is configured to provide, when the system is worn by a user, mechanical energy during a transition between a standing position and sitting position of the user.

Indeed, for unassisted prosthesis, the user must lift its whole weight using the force generated by its arms and the remaining natural leg. Thanks to the assistance by the actuator, the force that must be generated by the user is decreased.

According to other advantageous aspect of the invention, the actuator comprises a slider configured to slide along a sliding axis having a fixed actuator angle with one of the shared links or singular links, the slider being at a fixed distance from a pivot point disposed along one of the shared links or singular links rotatable with respect to the one of the shared links or singular links having the fixed angle with the sliding axis.

Indeed, contrarily to a gearbox, using a slider reduces the total weight of the system while providing high peak torque.

According to other advantageous aspect of the invention, each of the singular links and the common links comprises a number N of rods parallel to each other and fixed together by a locking axis parallel to the common rotation axis, N being greater than or equal to two, the locking axis locking the fixed rods in rotation, the femoral stem being fixed the one of the singular links along the corresponding locking axis.

Indeed, using several rods for each link enhance rigidity in a direction perpendicular to the functional plane.

According to other advantageous aspect of the invention, the femoral stem is configured to rotate around a remote rotation center, the anchoring element being joined to one of the four singular links of the double parallelogram mechanism, said singular link defining a reference axis, the femoral stem and the remote rotation center being on either side of the reference axis.

The anchoring element may thus be anchored closer to the bottom of the receiving shell thereby bringing the double parallelogram mechanism towards the back of the user so that the system does not protrude forward the user's body when anchored to a receiving shell and worn by the user.

According to other advantageous aspect of the invention, the shared link hinged to the reference axis and the shared link hinged to the singular link fixed to the femoral stem always have an angle lower than 90°.

This invention also relates to an external hip disarticulation prosthesis comprising the system for an external hip disarticulation prosthesis according to any one of claims 1 to 11 and a receiving shell, the anchoring element and the receiving shell being connected by the unique connection.

This allows to place the remote rotation close to the actual cotyle place while reducing the size of the system which therefore does not protrude forward the user's body.

According to other advantageous aspect of the invention, the femoral stem is configured to rotate around a remote rotation center located inside a user when the external hip disarticulation prosthesis is worn by the user.

This allows to place the remote rotation center close to the hip natural center of rotation (the actual position of the cotyle).

According to other advantageous aspect of the invention, one of the singular links of the double parallelogram mechanism is rigidly jointed to the receiving shell, the receiving shell overlaying the two extremities of said singular link when the external hip disarticulation prosthesis is worn by a user.

Therefore, the system does not protrude forward the user's body when anchored to a receiving shell and worn by the user thereby providing a better aesthetic.

According to other advantageous aspect of the invention, when the external hip disarticulation prosthesis is worn by a user seated on a surface, the receiving shell is contacting the surface, the double parallelogram mechanism being deported from underneath the receiving shell and being remote from the surface.

This allows a comfortable seated position and enhance usability thanks to the clearance below the receiving shell.

### DEFINITIONS

In the present invention, the following terms have the following meanings:
**"Distal"** refers to the position of an element farther from the receiving shell compared to another element or to the other elements of the prosthesis.
**"Hinging"** refers to a 1-degree rotational coupling between two elements. The two elements are thus rotationally jointed.
**"Joining"** refers to a coupling between two elements. The coupling may be a rigid joint or a rotational joint (a hinge).
**"Proximal"** refers to the position of an element closer to the receiving shell compared to another element or to the other elements of the prosthesis.
**"Remote rotation center"** refers to a rotation center located away from the prosthesis.
**"Functional plane"** refers to the plane wherein the kinematics of the prosthesis occurs. When the prosthesis is worn by a user, the functional plane may correspond to an anatomical plane (a sagittal plane) that divides the body into right and left sections and the plane wherein the femoral stem rotates may be a para-sagittal plane dividing the body of the user into unequal sections.

### DETAILED DESCRIPTION

The following detailed description will be better understood when read in conjunction with the drawings. For the purpose of illustrating, the system is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

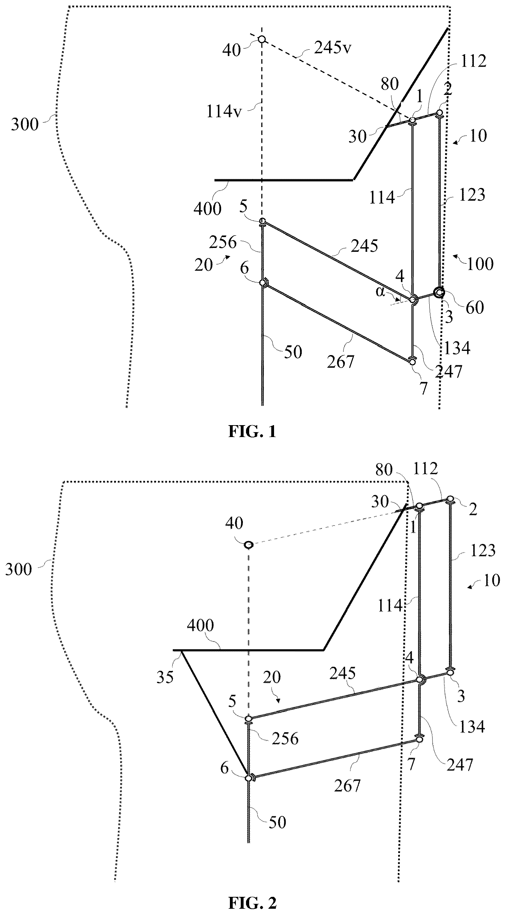

This invention relates to a system 100 for an external hip disarticulation prosthesis comprising a femoral segment configured to be connected with a receiving shell. The invention also relates to an external hip disarticulation prosthesis comprising the system 100 for an external hip disarticulation prosthesis connected to a receiving shell 400. A schematic representation of the invention is shown in figures 1, 3 and 4.

The system 100 is connected to the receiving shell 400 by an anchoring element 30 comprised in the system 100 and configured to provide a connection with the receiving shell 400.

The femoral segment comprises a double parallelogram mechanism, an actuator 60 configured to fold the double parallelogram mechanism and a femoral stem 50 connected to the double parallelogram mechanism. The femoral segment is thus composed of a set of elements and structures connected together and disposed between the knee (one of the extremities of the femoral stem) and the hip (the receiving shell) of the user. The connected elements and structures thus define connection path between the knee and the hip of the user. For a better understanding of the invention, the different elements and structures may be qualified as proximal when positioned close to the receiving shell 400 along the connection path and distal when positioned away from the receiving shell 400 (or close to the knee of the user) along the connection path.

A double parallelogram mechanism comprises two parallelogram structures: a proximal parallelogram structure 10 and a distal parallelogram structure 20.

Each parallelogram structure (10, 20) comprises four links. The four links are joined two by two at an intersecting point.

For clarity reasons, the links are numbered according to the parallelogram structure to which it belongs and to the intersecting points it links. For example, the link linking the intersecting points 3 and 4 of the parallelogram structure 10 is numbered 134 whereas the link linking the intersecting points 5 and 6 of the parallelogram structure 20 is numbered 256.

The proximal parallelogram structure 10 and the distal parallelogram structure 20 share one of their intersecting points. In other words, one of the intersecting points of the proximal parallelogram structure 10 and one of the intersecting points of the distal parallelogram structure 20 are aligned and overlap. This particular intersecting point is associated to a common rotation axis 4. The common rotation axis 4 is a rotational joint (a hinge) allowing a rotation with a unique degree of freedom.

Consequently, two links (114, 134, 247, 245) for each parallelogram structure (10, 20) are joined at the common rotation axis 4. In order to transmit the forces between the receiving shell 400 and the extremity of the femoral stem 50 associated to the knee of the user, one of said two links (114, 134) of the proximal parallelogram 10 structure and one of said two links (247, 245) of the distal parallelogram structure 20 are fixed with respect to each other. In other words, the intersecting point between said fixed links is locked (or rigid) in rotation leading to a rigid joint. For example, in figure 1, the link 114 of the proximal parallelogram structure and the link 247 of the distal parallelogram structure are fixed with respect to each other whereas the link 134 of the proximal parallelogram structure and the link 245 of the distal parallelogram structure are fixed with respect to each other. Each pair of links that are fixed with respect to each other are shared between the two parallelogram structures and form a common link. For example, in figure 1, the link 114 of the proximal parallelogram structure and the link 247 of the distal parallelogram structure form a first common link whereas the link 134 of the proximal parallelogram structure and the link 245 of the distal parallelogram structure form a second common link. The two common links are free in rotation relatively to each other about the common rotation axis 4. Therefore, the angle between the links 114 and 134 may be modified thereby modifying the angle between the links 245 and 247 rigidly jointed to the links 114 and 134. In other words, the double parallelogram mechanism comprises two common links hinged together at the common rotation axis 4, the common rotation axis 4 being positioned along each common link, *i.e.,* between the extremities (1, 7 and 5, 3) of each common link, thereby dividing each common link into the two shared links (114, 134, 245, 247). Therefore, for each common link, one shared link (114, 134) belongs to the proximal parallelogram structure 10 while the other shared link (245, 247) belongs to the distal parallelogram structure 20.

Each intersecting point of the links of the parallelogram structures (10, 20) which does not define the common links is associated to a rotation axis (1, 2, 3, 5, 6, 7) allowing the modification of an angle between the two links hinged at said intersecting point. In other words, these intersecting points are hinge joints allowing a rotation with a unique degree of freedom. These links free in rotation are singular links (112, 123, 267, 256) since they only belong to one of the parallelogram structures (10, 20) without rigid connection with the links of the other parallelogram structure (20, 10). The double parallelogram mechanism thus further comprises four singular links: two singular links for each parallelogram structure.

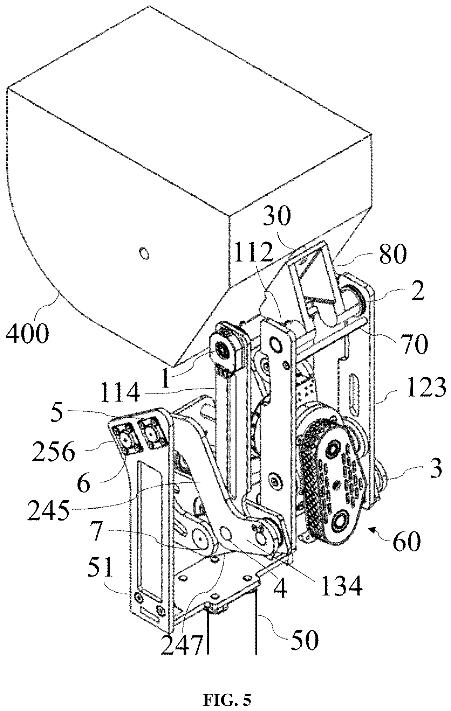

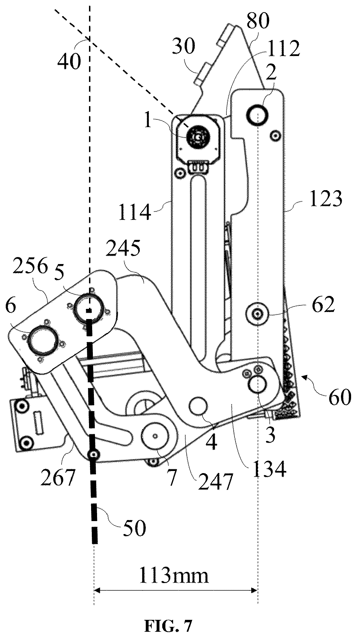

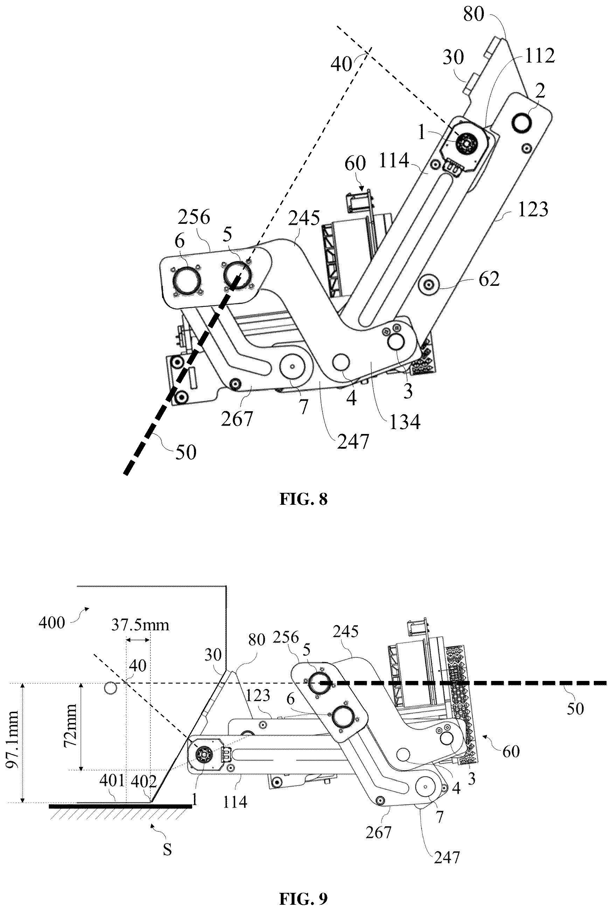

The modification of the angles between the links thus allows a folding and extension movement of the double parallelogram mechanism around the common rotation axis 4. The double parallelogram mechanism thus has a sitting configuration (illustrated in figure 9) wherein the double parallelogram mechanism is folded thereby allowing the sitting of a user wearing the system for an external hip disarticulation prosthesis. The double parallelogram mechanism also has a standing configuration (illustrated in figure 5 and 7) wherein the double parallelogram mechanism is partially extended thereby allowing the standing of a user wearing the system. The double parallelogram mechanism may be extended beyond the standing configuration to provide an extension movement to the user, the double parallelogram mechanism being in the extension configuration (illustrated in figure 8).

The kinematics of the double parallelogram mechanism is governed by the change of relative position between the two common links. The change of relative position corresponds to a rotation of one or the two common links around the common rotation axis 4. The folding and extension of the double parallelogram mechanism implies a rotation of the singular links around their rotation axis (1, 2, 3, 5, 6, 7). Therefore, the angle between the links at each rotation axis (1, 2, 3, 5, 6, 7) and the common rotation axis 4 is modified except for the angle between the shared links of the same common link.

The rotation axes (1, 2, 3, 5, 6, 7) preferably correspond to the vertices of the parallelogram structures (10, 20). The rotation axes (1, 2, 3, 5, 6, 7) are parallel to each other and parallel to the common rotation axis 4. Therefore, the kinematics of the double parallelogram mechanism is planar implying that the proximal parallelogram structure 10 and the distal parallelogram structure 20 are coplanar. In other words, the double parallelogram mechanism fold (bend) and extend parallelly to a functional plane perpendicular to the rotation axes, *i.e.,* with one degree of freedom.

The femoral segment is preferably configured to be anchored to the receiving shell 400 so that when the prosthesis is worn by a user, the functional plane corresponds to an anatomical plane that divides the user's body 300 into right and left sections. The connection between the anchoring element 30 and the receiving shell 400 may be a rigid connection. The rigid connection implies that there is no rotation or translation along the functional plane. Alternatively, a rotation along a plane different from the functional plane may be performed by the anchoring element 30 to reproduce abduction and adduction movements outside the sagittal plane of the user. The anchoring element 30 may be a direct rigid joint between one of the singular links (112, 123) of the proximal parallelogram structure 10 and the receiving shell 400. Preferably, the anchoring element 30 comprises a base 80 rigidly fixed to one of the singular links 112 of the proximal parallelogram structure 10 and the receiving shell 400. The base 80 may be aligned with said singular link as represented in figures 1 and 2 or may have a fixed angle with said singular link. Said singular link is preferably the singular link at the highest altitude when the system is worn by a user in a standing position, the altitude being defined along a vertical direction parallel to the gravity. Said singular link defines a reference axis which is the 112-axis delimiting the upper edge of the double parallelogram mechanism. The 112-axis is preferably inclined so that the rotation axis 2 distal to the rotation axis 1 is at higher altitude than the rotation axis 1 when the system is worn by a user in a standing position to avoid singular configurations.

The double parallelogram mechanism as described here-above allows to define two virtual parallelograms. Indeed, one of the shared links (245, 247) of a common link of the distal parallelogram structure 20 forms, with the shared link (114, 134) of the proximal parallelogram structure 10 and of the other common link, a virtual parallelogram. Indeed, each of said shared links defines a parallel virtual link (114v, 245v) joined to the other shared link at its rotation axis. For example, in figure 1, a main virtual parallelogram, represented by dashed lines, is formed by the link 245 of the distal parallelogram structure 20 and the link 114 of the proximal parallelogram structure 10. Indeed, the virtual link 245v parallel to the link 245 and jointed to the rotational axis 1 intersects the virtual link 114v parallel to the link 114 and jointed to the rotational axis 5 thereby forming with the shared links (114, 245) a parallelogram wherein only two links are material links. The intersecting point between the virtual links (114v, 245v) is the vertex of the main virtual parallelogram opposite the common rotational axis 4. The intersecting point of this main virtual parallelogram is remote from the double parallelogram mechanism since it is a rotational axis which does not correspond to one of the vertices of the distal parallelogram structure 20 or the proximal parallelogram structure 10. Said intersecting point is thus a remote rotation center 40. This remote rotation center 40 is important for the kinematics of the system since, as explained latter, it corresponds to the rotation axis of the femoral stem 50. Thanks to the double parallelogram mechanism, the remote rotation center 40 is thus located on the concave side of the receiving shell 400, *i.e.,* inside a user wearing the prosthesis. The exact position of the remote rotation center depends on the size of the links of the double parallelogram mechanism. Preferably, the remote rotation center 40 is located at the actual cotyle position.

It has to be noted that a secondary virtual parallelogram is formed by the link 247 of the distal parallelogram structure 20 and the link 134 of the proximal parallelogram structure 10. This secondary parallelogram does not define a rotational axis useful for the use of the external hip disarticulation prosthesis. However, in order to allow the folding of the double parallelogram mechanism until the sitting configuration, the angle of the vertex formed by the shared links defining the secondary virtual parallelogram must be large enough in the standing configuration, preferably larger than 90°. Indeed, since this angle reduces during the folding of the double parallelogram mechanism towards the sitting configuration, an angle lower than 90° implies a collapse of the shared links onto each other and blocks the folding of the double parallelogram mechanism.

An example of an external hip disarticulation prosthesis comprising a double parallelogram mechanism known from the state of art is shown in figure 2 and corresponds to the system disclosed in S. Luo et al. In this prior art prosthesis, the common links are straight. Therefore, the shared links (245 and 247) of the same common link are aligned. The common rotation axis may be the axis 4 as represented in figure 2 but may also be the axis 3, the common links being formed by the shared links 123 and 237 and by the links 245 and 134. The double parallelogram mechanism of the prior art prosthesis forms a main virtual parallelogram defining the remote rotation center 40. The remote rotation center 40 is aligned with the reference axis (112-axis) delimiting the upper edge of the double parallelogram mechanism, *i.e,* the remote rotation center 40 is aligned with the singular link 112. When the prior art system is anchored to a receiving shell 400 and worn by a standing user, the remote rotation center 40 is located at an altitude lower than the anchoring element 30 and lower than the singular link 112, the altitude being defined along a vertical direction parallel to the gravitational force. Therefore, in order to place the remote rotation center 40 close to the hip natural center of rotation (the actual position of the cotyle), the anchoring element 30 must be located at a higher vertical position than the cotyle and thus close to the edge of the receiving shell 400. The double parallelogram mechanism thus protrudes forward the user's body 300 and thus outside the receiving shell 400 and the natural volume of the leg of the user. This thus leads to an unaesthetic aspect of the prosthesis even when the user wears pants. Moreover, a second passive joint 35 with the receiving shell 400 is needed in this prior art prosthesis to provide constraints and supports for the stability in the stance phase of the gait. This second passive joint 35 is located on the underside of the receiving shell 400 which is uncomfortable when the user is sitting with the prosthesis.

To solve these problems, the double parallelogram mechanism of the invention has, for one of the common links, a fixed inclination angle α between the shared links of said common link as represented in figure 1. Said common link is thus an angled common link leading to an angled double parallelogram mechanism. In other words, the double parallelogram mechanism is tilted. The fixed inclination angle α is larger than 0°, *i.e.,* the shared links are not aligned. The fixed inclination angle α may range from 10° to 80°, preferably from 20° to 60°. For example, in figure 1, the shared links 245 and 134 have an inclination angle α of about 45°. This angled common link allows to bring the remote rotation center 40 up with respect to the double parallelogram mechanism while maintaining the angle of the vertex formed by the shared links (134, 247) defining the secondary virtual parallelogram larger than 90°. Therefore, the remote rotation center 40 - and thus the virtual link 245v parallel to the link 245 - is not aligned anymore with the 112-axis defining the upper edge of the double parallelogram mechanism. The angle of the vertex of the main virtual parallelogram corresponding to the remote rotation center 40 is always lower than 90°, i.e, whatever the configuration of the double parallelogram mechanism. This angle is the same as the angle between the shared link 114 and the shared link 245.

When the system 100 of the invention is anchored to a receiving shell 400 and worn by a standing user, the remote rotation center 40 is located at an altitude higher than the 112-axis. In other words, the femoral stem 50 and the remote rotation center 40 are on either side of the 112-axis. Therefore, while maintaining the remote rotation center 40 at a position close to the hip natural center of rotation, the double parallelogram mechanism is brought down and backwards compared to the prior art double parallelogram mechanism. The double parallelogram mechanism of the invention is thus placed below the user, i.e., at the natural position of the leg of the user instead of protruding forward the user's body 300. In other words, the receiving shell 400 overlays the two extremities of the singular link 112 defining the 112-axis. Moreover, the size of the distal parallelogram structure 20 may be reduced (more compact) compared to the prior art system while maintaining the remote rotation center 40 at a position close to the hip natural center of rotation.

Moreover, thanks to the invention, no second passive joint with the receiving shell 400 is needed to provide constraints and supports for the stability during the gait. In other words, the anchoring element 30 is configured to provide a unique connection with a receiving shell 400. This unique connection may be a rigid joint, preferably a rigid connection along a sagittal plane of the user. The anchoring element 30 may comprise several fasteners, for example 4 screws, but only one element of the system 100 is connected to the receiving shell 400 to provide the unique connection. This thus leads to a clear space below the receiving shell 400 when the user is sitting, as illustrated in figure 9. In other words, when the user wearing the system 100 is seated on a surface S, only the receiving shell 400 is contacting said surface S, the double parallelogram mechanism being deported from underneath the receiving shell 400 and being remote from said surface S.

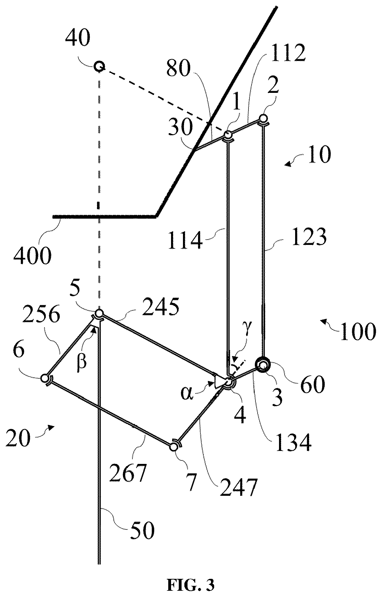

In a preferred embodiment, the shared links of each of the two common links have a fixed inclination angle (α, γ) between them as represented in figure 3. This advantageously avoids singularities in the relative positions of the links when passing from the sitting configuration to the standing configuration and vice-versa. Indeed, a singularity between two links requires a very large torque to change the angle between these links. Therefore, avoiding singularities allows to reduce the peak torque needed and thus the size and weight of the actuator 60. The two fixed inclination angles (α, γ) may differ from each other, their values are independently determined to optimize compactness and extension ranges and to avoid kinematic singularities. The main fixed inclination angle α may range from 10° to 80°, preferably from 20° to 60°. The secondary fixed inclination angle γ may range from 10° to 80°, preferably from 20° to 60°. As represented in figure 3 for example, the main inclination angle α between the shared links 245 and 134 is about 45° while the secondary fixed inclination angle γ between the shared links 114 and 247 is about 40°.

As explained above, the system 100 further comprises an actuator 60 configured to fold the double parallelogram mechanism around the common rotation axis4. The actuator 60 is thus configured to modify at least one moveable or unfixed angle between the links of the double parallelogram mechanism. Indeed, providing mechanical energy by applying a force or a torque to modify one angle of double parallelogram mechanism implies the transfer of that force or torque to the other rotational axes and the common rotational axis via the links and thus implies the folding or extension of the double parallelogram mechanism.

The actuator 60 may be configured to provide, when the system 100 is worn by a user, mechanical energy during a gait of the user.

The actuator 60 may be configured to provide, when the system 100 is worn by a user, mechanical energy during a transition from a standing position to a sitting position of the user. The actuator 60 may also be configured to provide, when the system 100 is worn by a user, mechanical energy during a transition from a sitting position to a standing position of the user. Finally, the actuator 60 may also be configured to provide, when the system 100 is worn by a user, mechanical energy during a transition between a sitting position and a standing position of the user, *i.e.,* from a standing position to a sitting position of the user and from a sitting position to a standing position of the user.

The actuator 60 may be configured to provide, when the system 100 is worn by a user, mechanical energy during a gait of the user and during a transition between a standing position and sitting position of the user.

The actuator 60 may comprise a non-linear transmission. The actuator 60 may be configured to provide a peak absolute torque, *i.e.* a maximum torque in absolute value, ranging from 100 Nm to 200 Nm. The torque may be either positive leading to the folding of the double parallelogram mechanism or negative leading to the extension of the double parallelogram mechanism.

In one embodiment, the actuator 60 is a motor, preferably a rotational motor, comprised in or around one of the rotation axes as in figures 1 and 3.

In a preferred embodiment, the actuator 60 is connected to two links moveable with respect to each other.

In a first variant of the preferred embodiment, the two links may be parallel links of the same parallelogram structure. The actuator 60 is therefore configured to modify the distance between the links. For example, shortening the distance between the link 245 and 267 implies folding the double parallelogram mechanism while increasing the distance between the link 245 and 267 implies extending the double parallelogram mechanism. The actuator 60 comprises two sliders configured to slide along one of the shared links (114, 134, 247, 245) or singular links (112, 123, 267, 256). The two sliders are configured to slide along the same link. Each slider is at the same fixed distance from a pivot point disposed along the shared link (114, 134, 247, 245) or singular link (112, 123, 267, 256) parallel to said link onto which the sliders are configured to slide. The pivot points have a single axis of rotation parallel to the rotation axes of the double parallelogram mechanism (i.e. perpendicular to the functional plane). The distance between the slider and the pivot point may be kept constant thanks to a solid link. The solid link may be straight or curved along the functional plane. The sliders and the pivot points form thus an isosceles trapezium or a parallelogram. Therefore, sliding the sliders on the same direction in case of a parallelogram or in opposite directions in case of an isosceles trapezium implies reducing or increasing the distance between the links comprising the sliders and the pivot points and thus the angle at the rotation axes of these links. The slider may comprise a direct current (DC) motor. The slider may slide outside the link or inside the link. Alternatively, the actuator 60 comprises two pairs of pivot points. One pivot point of each pair of pivot points is disposed along the shared link (114, 134, 247, 245) or singular link (112, 123, 267, 256) while the other pivot point of said pair of pivot points is disposed along the parallel shared link (114, 134, 247, 245) or singular link (112, 123, 267, 256). The four pivot points are disposed so that to form an isosceles trapezium or a parallelogram. The variable link may be straight or curved along the functional plane. The variable links have the same length. Reducing or increasing the length of the variable length links by the same amount implies reducing or increasing the distance between the links comprising the pivot points.

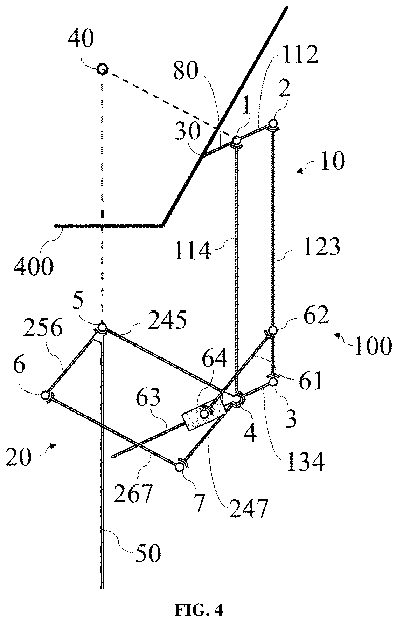

In a second variant of the preferred embodiment, the two links may be consecutive links jointed at a rotational axis. The actuator 60 is therefore configured to modify the angle between these two links by moving them closer or further apart. The actuator 60 comprises one slider 64 configured to slide along a sliding axis 63. An example of the second variant of the preferred embodiment is represented in figure 4. The sliding axis 63 has a fixed actuator angle with one of the shared links (114, 134, 247, 245) or singular links (112, 123, 267, 256). The fixed actuator angle may be equal to 0° as in figure 4. The fixed actuator angle equal to 0° leads to the sliding axis 63 aligned with one of the links. The sliding axis 63 may be superimposed on said link or may extend said link. The slider 64 may thus slide between or outside the intersecting points at which said link is joined. The sliding axis 63 may be longer than said link, shorter than said link. The slider 64 is at a fixed distance from a pivot point 62 disposed along one of the shared links (114, 134, 247, 245) or singular links (112, 123, 267, 256) rotatable (*i.e.,* not parallel) with respect to said link having the fixed angle with the sliding axis 63. The pivot point 62 has a single axis of rotation parallel to the rotation axes of the double parallelogram mechanism (i.e. perpendicular to the functional plane). The distance between the slider 64 and the pivot point 62 may be kept constant thanks to a solid link 61. The solid link 61 may be straight or curved along the functional plane. The actuator 60 is configured to modify the angle between the sliding axis 63 and the link comprising the pivot point 62 by moving them closer or further apart. Indeed, the direction of the sliding axis 63, the direction of the link comprising the pivot point 62, and the direction of the slider 64 form a triangle. Therefore, sliding the slider 64 implies modifying the angle between said directions and thus the angle between the link having the fixed actuator angle with the sliding axis 63 and the link comprising the pivot point 62. The slider may comprise a direct current (DC) motor. The slider may slide around the sliding axis 63 or inside the sliding axis 63. Alternatively, the actuator 60 comprises two pivot points. The two pivot points are disposed along the shared links (114, 134, 247, 245) or singular links (112, 123, 267, 256) rotatable (*i.e.,* not parallel) with respect to each other. The pivot points are linked by a variable length link. The pivot points form, with directions of the links onto which the pivot points are disposed, a triangle. The variable link may be straight or curved along the functional plane. Reducing or increasing the length of the variable length link implies modifying the angle between said directions and thus a rotation of the links comprising the pivot points.

As explained above, the system 100 further comprises a femoral stem 50. The femoral stem 50 is rigidly jointed to one of the singular links 256. The femoral stem 50 is configured to rotate around the remote rotation center 40. The plane wherein the femoral stem rotates may be, when a user wears the system 100, a para-sagittal plane dividing the body 300 of the user into unequal sections. The femoral stem 50 may be aligned with the singular link 256 - figure 1 - or may have a femoral angle β with the singular link 256 - figure 3. The femoral angle β may be a fixed angle ranging from 10° to 80° as represented in figure 3. The angle between the femoral stem 50 and the singular link may be configured so that, when the system 100 is worn by a standing user, the femoral stem 50 is vertical. Preferably, the femoral stem 50 is parallel to the shared link 114 joining the reference axis of the proximal parallelogram structure 10.

The description hereabove is a description of the global kinematics of the system 100 wherein the overall elements have no thickness and lies within a unique functional plane. However, in the actual system 100, each element has a thickness. Moreover, each link may be curved in the functional plane or outside the functional plane, or each link may change its thickness. For example, two non-consecutive links of one parallelogram structure may be curved in a complementary direction in the functional plane such as the links 245 and 267 shown in figure 7. The curvature allows to increase the compactness of the system 100. Moreover, the curved links may be in the same functional single plane thereby facilitating the mechanical manufacturing. Finally, the curvature also allows to define one or more mechanical stop during the folding or extension of the double parallelogram mechanism.

Furthermore, the joining between the links implies a superposition of the links thereby increasing the total thickness of the double parallelogram mechanism. Therefore, each parallelogram structure (10, 20) has a thickness measured along the rotation axes.

In one embodiment, each link comprises a number N of rods parallel to each other along the direction of the rotation axes. The rods preferably have mirror shapes. The rods of one link may be fixed together by a locking axis 70 parallel to the rotation axes. The locking axis 70 locks the rods fixed to it in rotation. In other words, the rotation of the link corresponds to the simultaneous and identical rotation of all the rods of this link. The femoral stem 50 is preferably centered along the locking axis 70 of the singular link to which it is fixed. The actuator 60 is preferably positioned between the rods.

The material used to manufacture the links and the locking axis may be any material configured to resist to the forces generated by the actuator 60 and the weight of the user. For example, a suitable material is titanium or aluminum alloy. The preferred material is aluminum thanks to its high resistance and light density.

### BRIEF DESCRIPTION OF THE DRAWINGS

**Figure 1** is a schematic representation of the external hip disarticulation prosthesis according to one embodiment superimposed on a silhouette of a user's body.
**Figure 2** is a schematic representation of a prior art external hip disarticulation prosthesis superimposed on a silhouette of a user's body.
**Figure 3** is a schematic representation of the external hip disarticulation prosthesis according to an embodiment wherein the two common links are angled.
**Figure 4** is a schematic representation of the external hip disarticulation prosthesis according to an embodiment wherein the actuator comprises a slider.
**Figure 5** is a three-dimensional 3/4 view of the external hip disarticulation prosthesis according to an embodiment wherein the two common links are angled and the actuator comprises a slider. Standing configuration.
**Figure 6** is a three-dimensional backside view of the system for the external hip disarticulation prosthesis of figure 5.
**Figure 7** is a three-dimensional profile view of the system of figure 6 in the standing configuration. For clarity, femoral stem 50 and U-shaped element 51 are omitted.
**Figure 8** is a three-dimensional profile view of the system of figure 6 in the extension configuration. For clarity, femoral stem 50 and U-shaped element 51 are omitted.
**Figure 9** is a three-dimensional profile view of the external hip disarticulation prosthesis of figure 5 in the sitting configuration. For clarity, femoral stem 50 and U-shaped element 51 are omitted.
**Figure 10** is a three-dimensional profile view of the actuator comprising a slider.

### EXAMPLE

The present invention is further illustrated by the following example describing a prototype of an external hip disarticulation prosthesis according to the invention.

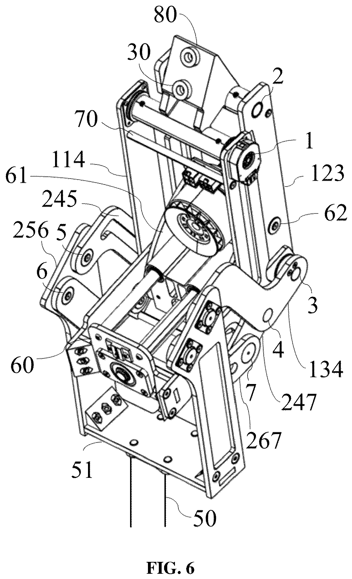

The system 100 of this example is represented in figures 5 to 10.

The dimensions of the system are determined to provide a remote rotation center 40 at a distance of 97.1 mm inside the receiving shell 400 from the bottom 401 of the receiving shell 400 and at a distance of 37.5 mm from the bottom corner 402 of the receiving shell 400 as represented in figure 9.

The system is shown in the standing configuration (figure 7), in the extension configuration (figure 8) and in the sitting configuration (figure 9).

The link 114 of the proximal parallelogram structure 10 and the link 247 of the distal parallelogram structure 20 form the first common link whereas the link 134 of the proximal parallelogram structure 10 and the link 245 of the distal parallelogram structure 20 form the second common link. Each common link is formed in one piece thereby increasing the robustness of the system 100.

The links 245 and 267 of the distal parallelogram structure 20 are curved along the functional plane thereby forming a L-shape. This L-shape defines a mechanical stop which limits the range of extension angle.

The shared link 247 has a length of 35 mm while the singular link 123 has a length of 160 mm. The length of a link is measured between the two intersecting points to which said link is joined.

The link 112 defining the reference axis is angle by 20.5° compared to a horizontal direction when the prosthesis is worn by a user. The rotation axis 2 is at higher altitude than the rotation axis 1.

Each link comprises two rods locked together by a locking axis 70 parallel to the rotation axes. The rods are flat bars, i.e, with the same thickness along the functional plane. The rods are aluminum bars - AW7075-T651 alloy.

The femoral stem 50 is centered along the locking axis 70. The centering and fixing of the femoral stem 50 to the singular link 256 is allowed by a U-shaped element 51 manufactured in one piece, and thus rigidly fixed to both rods constituting the singular link 256. The femoral stem 50 have a fixed femoral angle β of 55° with the singular link 256 so that when the system 100 is worn by a standing user, the femoral stem 50 is vertical. The femoral stem 50 is parallel to the shared link 114 joining the reference axis of the proximal parallelogram structure 10.

The anchoring element 30 comprises a base 80 manufactured in one piece with the singular link 112 to connect it to the receiving shell 400. The intersection between the 112-axis and the receiving shell 400 is at a vertical distance of 72 mm below the remote rotation center 40 when the system 100 is worn by a user as represented in figure 9. The remote rotation center 40 is thus brought up relatively to the 112-axis thanks to the angled double parallelogram mechanism. The length of the 112-axis s measured from the receiving shell 400 to the rotational axis 2 is 67 mm. The 112-axis is inclined by 110.5° with respect to the direction of the femoral stem 50 in the standing configuration.

The main fixed inclination angle α between the shared links 245 and 134 is 60° while the secondary fixed inclination angle γ between the shared links 114 and 247 is 55°. The femoral stem 50 is thus parallel to the shared link 114.

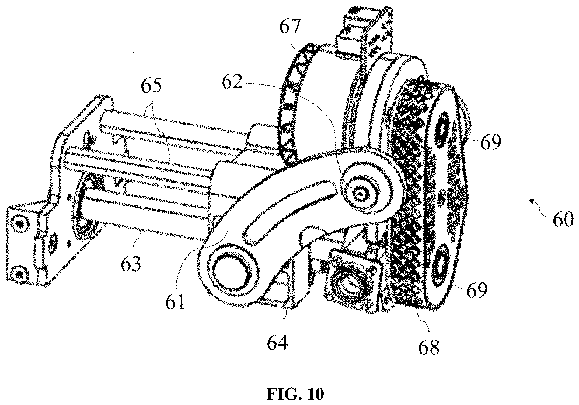

The actuator 60, better shown in figure 10, is configured to provide, when the system 100 is worn by a user, mechanical energy during a gait of the user and during a transition between a standing position and sitting position of the user.

The actuator 60 comprises one slider 64 configured to slide along a sliding axis 63. The sliding axis 63 has a fixed actuator angle of 15° with the shared link 134. The slider 64 is at a fixed distance of 47 mm from the pivot point 62 disposed along the singular link 123 at 47 mm from the rotational axis 3. The sliding axis 63 is a power screw coupled to a motor 67 via a belt and two pulleys. The belt may be protected by a belt cover 68 represented for example in figure 10. Each pulley may be mounted on a bearing 69 as represented in figure 10. The power screw has a length of 136 mm. The actuator 60 further comprises two guiding rails 65 to stabilize the sliding of the slider 64. The motor 67 is a direct current (DC) motor - *EC 60 fat 200W 48V* manufactured by Maxon, Sachseln. This actuator 60 advantageously provide a high transmission ratio and therefore high torque when it is the most needed; *i.e.,* at the beginning of the single support phase.

The total width of the double parallelogram mechanism measured in the standing configuration along the functional plane from the rotational axis 5 to the rotational axis 2 perpendicularly to direction of the femoral stem 50 is 113 mm as shown in figure 7. The total thickness of the system 100 is 136 mm. The height of the double parallelogram mechanism measured from the remote rotation center 40 to the bottom of the U-shaped element 51 in the standing configuration is 380 mm. The U-shaped element 51 is not represented in figure 7 for clarity reasons.

This size can be compared to the size of the prior art system of S. Luo et al. presented in figure 2. The total width of the prior art double parallelogram mechanism is 142 mm. The total thickness of the prior art system is 146 mm. The height of the prior art double parallelogram mechanism measured from the remote rotation center 40 to the bottom of the U-shaped element 51 is 415 mm.

The system 100 of the invention allows a flexion angle of 90° (the sitting configuration of figure 9) and an extension angle of 30° (the extension configuration of figure 8). The extension angle corresponds to the rearmost position of the leg during the gait. As shown in figure 9, the bottom of the receiving shell 400 is clear of any element of the system thereby providing a direct contact between the receiving shell 400 and a surface S wherein the user wearing the prothesis is sit.

The system is conceived to have a life time of 5 years, and allows a user to perform daily 4000 steps and 71 transitions between the standing configuration, sitting configuration and standing configuration.

### NUMERICAL REFERENCES

100 - System for an external hip disarticulation prosthesis // 1, 2, 3, 5, 6, 7 - Rotation axes // 4 - Common rotation axis // 10 - Proximal parallelogram structure // 20 - Distal parallelogram structure // 112, 123 - Singular links of the proximal parallelogram structure // 114, 134 - Shared links of the proximal parallelogram structure // 245, 247 - Shared links of the distal parallelogram structure // 267, 256 - Singular links of the distal parallelogram structure // 30 - Anchoring element // 35 - Second passive joint // 40 - Remote rotation center // 245v, 114v - Virtual links of the main virtual parallelogram // 50 - Femoral stem // 51 - U-shaped element // 60 - Actuator // 61 - Solid link // 62 - Pivot point // 63 - Sliding axis // 64 - Slider // 65 - Guiding rails // 67 - Motor // 68 - Belt cover // 69 - Bearing // 70 - Locking axis // 80 - Base // 300 - User's body // 400 - Receiving shell // 401 - Bottom of the receiving shell // 402 - Bottom corner of the receiving shell // S - Surface wherein the user is seated // α - Main fixed inclination angle // β - Femoral angle // γ - Secondary fixed inclination angle

## Claims

1. A system (100) for an external hip disarticulation prosthesis comprising:
- A double parallelogram mechanism comprising four singular links (112, 123, 267, 256) and two common links, each of the singular links (112, 123, 267, 256) and common links comprising two extremities,
the two common links joining together at a common rotation axis (4), the common rotation axis (4) being positioned between the extremities of each common link thereby dividing each common link into two shared links (114, 134, 245, 247), and
for at least one common link, the shared links (114, 134, 245, 247) of said common link being angled by a fixed non-zero inclination angle (α, γ),
- An actuator (60) configured to fold the double parallelogram mechanism around the common rotation axis (4), and
- A femoral stem (50) fixed to one of the singular links (112, 123, 267, 256),
the double parallelogram mechanism having a planar kinematics,
the double parallelogram mechanism comprising an anchoring element (30) configured to provide a unique connection with a receiving shell (400).

2. The system (100) for an external hip disarticulation prosthesis according to claim 1, wherein the femoral stem (50) has a femoral angle (β) with the singular link (112, 123, 267, 256) to which it is fixed, the femoral angle (β) being a fixed angle ranging from 10° to 80°.

3. The system (100) for an external hip disarticulation prosthesis according to claim 1 or 2, wherein the unique connection between the anchoring element (30) and the receiving shell (400) is a rigid connection.

4. The system (100) for an external hip disarticulation prosthesis according to any one of claims 1 to 3, wherein for each angled common link, the fixed inclination angle (α, γ) is ranging from 10° to 80°.

5. The system (100) for an external hip disarticulation prosthesis according to any one of claims 1 to 4, wherein the actuator (60) is configured to provide a peak absolute torque ranging from 100 Nm to 200 Nm.

6. The system (100) for an external hip disarticulation prosthesis according to any one of claims 1 to 5, wherein the actuator (60) is configured to provide, when the system (100) is worn by a user, mechanical energy during a gait of the user.

7. The system (100) for an external hip disarticulation prosthesis according to any one of claims 1 to 6, wherein the actuator (60) is configured to provide, when the system (100) is worn by a user, mechanical energy during a transition between a standing position and sitting position of the user.

8. The system (100) for an external hip disarticulation prosthesis according to any one of claims 1 to 7, wherein the actuator (60) comprises a slider (64) configured to slide along a sliding axis (63) having a fixed actuator angle with one of the shared links (114, 134, 245, 247) or singular links (112, 123, 267, 256), the slider (64) being at a fixed distance from a pivot point (62) disposed along one of the shared links (114, 134, 245, 247) or singular links (112, 123, 267, 256) rotatable with respect to the one of the shared links (114, 134, 245, 247) or singular links (112, 123, 267, 256) having the fixed angle with the sliding axis (63).

9. The system (100) for an external hip disarticulation prosthesis according to any one of claims 1 to 8, wherein each of the singular links (112, 123, 267, 256) and the common links comprises a number N of rods parallel to each other and fixed together by a locking axis (70) parallel to the common rotation axis (4), N being greater than or equal to two, the locking axis (70) locking the fixed rods in rotation, the femoral stem (50) being fixed the one of the singular links (112, 123, 267, 256) along the corresponding locking axis (70).

10. The system (100) for an external hip disarticulation prosthesis according to any one of claims 1 to 9, wherein the femoral stem (50) is configured to rotate around a remote rotation center (40), the anchoring element (30) being joined to one of the four singular links (256) of the double parallelogram mechanism, said singular link (256) defining a reference axis, the femoral stem (50) and the remote rotation center (40) being on either side of the reference axis.

11. The system (100) for an external hip disarticulation prosthesis according to any one of claims 1 to 10, wherein the shared link (114) hinged to the reference axis and the shared link (245) hinged to the singular link fixed to the femoral stem (50) always have an angle lower than 90°.

12. An external hip disarticulation prosthesis comprising the system (100) for an external hip disarticulation prosthesis according to any one of claims 1 to 11 and a receiving shell (400), the anchoring element (30) and the receiving shell (400) being connected by the unique connection.

13. The external hip disarticulation prosthesis according to claim 12, wherein the femoral stem (50) is configured to rotate around a remote rotation center (40) located inside a user when the external hip disarticulation prosthesis is worn by the user.

14. The external hip disarticulation prosthesis according to claim 12 or 13, wherein one of the singular links (112, 123, 267, 256) of the double parallelogram mechanism is rigidly jointed to the receiving shell (400), the receiving shell (400) overlaying the two extremities of said singular link (112, 123, 267, 256) when the external hip disarticulation prosthesis is worn by a user.

15. The external hip disarticulation prosthesis according to any one of claims 11 to 14, wherein, when the external hip disarticulation prosthesis is worn by a user seated on a surface (S), the receiving shell (400) is contacting the surface (S), the double parallelogram mechanism being deported from underneath the receiving shell (400) and being remote from the surface (S).
